# EUROPEAN PATENT APPLICATION

(11) **EP 1 946 747 A1**
(43) Date of publication of application: **23.07.2008**
(21) Application number: 07100681.1
(22) Date of filing: 17.01.2007
(51) Int. Cl.: A61K 9/08, A61K 47/26, A61K 9/00, A61K 31/00

(54) **Pharmaceutical composition of improved stability containing taxane derivatives**

(71) Applicant: SANDOZ AG, 4056 Basel (CH)
(72) Inventor: The designation of the inventor has not yet been filed
(74) Representative: Barbier, Denis Robert

(57) **Abstract**

Novel stable taxane derivatives based compositions comprising polysorbate 80 non-standard grades. The addition of an organic and/or an inorganic acid can further increase the stability of the composition.

## Description

The present invention relates to pharmaceutical compositions with improved stability comprising taxane derivatives, especially docetaxel or paclitaxel, and polysorbate 80 non-standard grades.

Taxane derivatives, such as docetaxel and paclitaxel are well known and established drugs in the treatment of malignant tumours. For example docetaxel is marketed by Sanofi-Aventis under the trade name Taxotere^{®} and paclitaxel is marketed by Bristol-Myers-Squibb under the trade name Taxol^{®}. The low solubility of docetaxel and paclitaxel are well documented and is the major cause for preparation of the composition for injection containing surfactant. EP 0 593 601, EP 0 593 656 and EP 0 672 912 describe the use of polysorbates, polyoxyethylene glycol esters and polyethoxylated castor oils as suitable surfactants.

The currently marketed composition of Taxotere^{®} comprises a two compartment formulation composed of a vial containing a solution of Docetaxel in polysorbate 80 and a solvent vial containing an aqueous solution of ethanol. This two compartment system makes the handling for the pharmacist complicated. The system requires two dilutions prior to administration to the patient. The drug vial has prior to use to be reconstituted with the solvent vial making sure that the polysorbate 80 is properly reconstituted but without significant foaming. This solution then has to be further diluted by injection of the appropriate amount of solution into an infusion bag. The currently marketed composition contains an overfill for both the drug vial and the solvent vial. Thus, apart from the handling this implies a risk for the proper dosing to the patient.

One compartment compositions contain a solution of the drug in a single vial with a well known concentration of the solution. This solution has to be withdrawn from the vial and injected into the infusion bag. As the vial does not have to be reconstituted and homogenized prior to use there is no risk of foaming.

EP 593 656 (Rhone-Poulenc Rorer) describe a one compartment composition containing a taxane derivative in association with ethanol and Polysorbate 80. However this composition proved to be fairly unstable. This composition shows a significant degradation, expressed in the formation of 7-Epi-docetaxel when exposing it to heating. 7-Epi-docetaxel is well a known degradation product of the Docetaxel (for example see Vasu Dev et al., J. pharm. biomed. anal. 40 (2006), 614-622)

It would be therefore highly desirable to develop alternative compositions having an improved stability.

The Applicant has now surprisingly found that the stability of taxane derivatives containing compositions can be significantly improved when using non-standard grades of polysorbate 80.

Standard grades of polysorbate 80 comply with the limits as laid down in the European Pharmacopoeia 2007. Those standard grades may and do contain water up to 3% and peroxide values of up to 10 (see European Pharmacopoeia 2007, part 5.4).

According to the present invention a polysorbate 80 standard grades contains water up to 3% and peroxide values of up to 10. Such standard grades are for example commercialized by Croda under the trademark Crillet NF^{®},

### (http://www.crodausa.com/html product sheets/PHfeatureCrills2.htm).

According to the present invention a polysorbate 80 non-standard grades has a water content of less than 1.5%, preferably 0.5%, more preferably less that 0.2% and a peroxide value of less than 5, preferably less than 3, more preferably less than 2. Such non-standard grades are for example commercialized by Croda under the trademark Crillet 4 HP^{®}. Those grades still comply to the European Pharmacopoeia but they are considered as non-standard grades as they comply to much tighter limits than required by the European Pharmacopoeia.

The water content of the polysorbate 80 is measured by Karl- Fischer analysis as laid down in the European Pharmacopoeia 2007.

The peroxide value of the polysorbate 80 is measured by a method as laid down in the European Pharmacopoeia 2007 (see European Pharmacopoeia 2007, part 5.4).

The object of the present invention is related to a pharmaceutical composition comprising
- a pharmaceutical acceptable taxane derivative of formula (I) 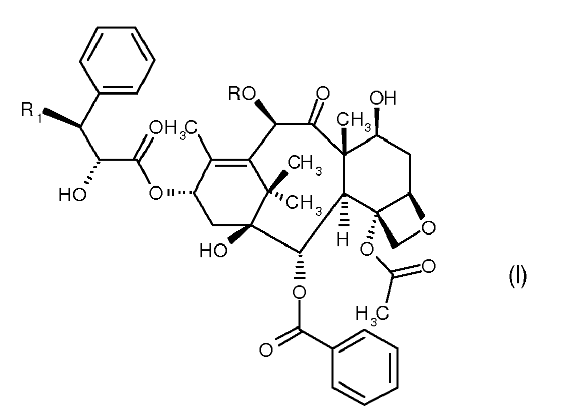 wherein R represents a hydrogen atom or an acetyl radical and R₁ represents a terbutoxycarbonylamino or a benzoylamino radical, and
- a polysorbate 80 having a water content of less than 1.5%, preferably less than 0.5%, more preferably less than 0.2 % and a peroxide value of less than 5, preferably less than 3, more preferably less than 2.

Preferably, the composition according to the invention further contains a physiologically acceptable solvent. More preferably, the solvent is selected in the group consisting of ethanol, DMSO, polyethylene glycol, glycerol, propanediol, polypropylene glycol, glycofurol, benzyl alcohol and N-methyl-2-pyrolidone. Most preferred solvent is ethanol. Especially preferred solvent is ethanol anhydrous as defined in the European Pharmacopoeia 2007.

Preferably, the taxane derivative of formula (I) is selected from the group consisting of docetaxel and paclitaxel. More preferred taxane derivative of formula (I) is docetaxel.

According to the present invention the taxane derivative of formula (I) is in form of a pharmaceutically acceptable anhydrate, solvate and/or hydrate.

Moreover, it is known that Taxane derivates show improved stability in acidic pH ranges. For example EP 0 835 657 A1 describes the use of citric acid to stabilize Paclitaxel by adjusting the pH into the range 1 to 8, preferably 5 to 7. WO 03/053350 describes the use of citric acid in Taxane compositions containing solubilizing compounds such as polyethylene glycols or surfactants which have residual levels of alkali metals. The pH values of the compositions presented in WO 03/053350 are not explicitly mentioned.

Commercial Docetaxel compositions and/or compositions described in EP 0 593 656 tend to show an acidic pH per se.

Taxotere as available on the market (batch number 4D405) shows a pH of 4.0 after dilution at a rate of 1:54 with 0.9% saline. The final concentration of this solution was 0.74 mg/ml Docetaxel.

Solutions prepared according to EP 0 593 656, prepared with either polysorbate 80 standard grades or with polysorbate 80 non-standard grades show a pH of 5.2 after dilution at a rate of 1:27 with 0.9% saline. The final concentration of this solution was 0.74 mg/ml Docetaxel.

It has surprisingly been found by the Applicant that even under these acidic conditions, the addition of an organic and/or inorganic acid induces a significant increase in stability of the composition according to the present invention.

Accordingly, in a preferred embodiment, the composition according to the invention as previously described further contains an organic and/or and inorganic acid. Preferably the acid is selected in the group consisting of citric acid, acetic acid, formic acid, ascorbic acid, aspartic acid, benzoic acid, hydrochloric acid, sulphuric acid, phosphoric acid, tartaric acid, glutamic acid, lactic acid, maleic acid or succinic acid. Most preferred acid is citric acid. Especially preferred acid is citric acid anhydrous as defined in the European Pharmacopoeia 2007.

Preferably, the concentration of citric acid is from 0.5 to 2 mg/ml and/or where citric acid is added in a ratio (mg citric acid : mg Docetaxel) of from 1:40 to 1:10.

A preferred embodiment according to the present invention is a pharmaceutical composition compris ing in a one or two compartment system
- a taxane derivative of formula (I) as previously described, especially docetaxel,
- a polysorbate 80 having a water content of less than 1.5%, preferably less than 0.5%, more preferably less than 0.2 % and a peroxide value of less than 5, preferably less than 3, more preferably less than 2,
- ethanol, especially ethanol anhydrous as defined in the European Pharmacopoeia 2007 and,
- citric acid, especially citric acid anhydrous as defined in the European Pharmacopoeia 2007.

A more preferred embodiment according to the present invention is to a pharmaceutical composition comprising in a one or two compartment system, preferably in a one compartment system:
- docetaxel,
- a polysorbate 80 having a water content of less than 1.5%, preferably less than 0.5%, more preferably less than 0.2 %, and a peroxide value of less than 5, preferably less than 3, more preferably less than 2,
- ethanol anhydrous as defined in the European Pharmacopoeia 2007 and,
- citric acid anhydrous as defined in the European Pharmacopoeia 2007.

Preferably, the water content of the composition according to the invention is less than about 1.5% by weight of the composition, especially less than about 0.5% by weight of the composition.

This invention will be better understood from the examples that follow. However, these examples illustrate but do not limit the invention. Those skilled in the art will readily appreciate that the specific process and results discussed are merely illustrative of the invention as described more fully in the claims that follow thereafter.

The following non-limiting examples illustrate further aspects of the invention.

### Example 1: Manufacturing process of a composition according to the invention - with polysorbate non standard grade (Crillet 4 HP)

Dissolve 1.8 g of Docetaxel in approximately 30 ml of ethanol absolute by stirring at ambient temperature. Add 46.8 g of polysorbate 80 non standard grade (Crillet 4 HP) to the solution and stir to get a homogenous solution. Fill up to 90 ml with ethanol absolute and mix. Filter the solution and fill the solution into vials. Flush the vials with nitrogen. Close the vials with flouropolymer coated rubber stoppers and seal them with crimp caps.

### Example 2: Manufacturing process of a composition with polysorbate standard grade (Crillet NF)

Dissolve 1.8 g of Docetaxel in approximately 30 ml of ethanol absolute by stirring at ambient temperature. Add 46.8 g of polysorbate 80 (Crillet NF) to the solution and stir to get a homogenous solution. Fill up to 90 ml with ethanol absolute and mix. Filter the solution and fill the solution into vials. Flush the vials with nitrogen. Close the vials with flouropolymer coated rubber stoppers and seal them with crimp caps.

### Example 3: Comparative Thermal test

The sealed vials manufactured according to examples 1 and 2 are put into a thermostatic water bath at 70° C. The vials are shaken every 10 minutes. The vials are stored in the water batch protected from light. The vials are removed after 30 minutes, respectively 120 minutes from the water-bath and allowed to cool to room temperature. The vials are analyzed by HPLC for presence of 7-Epi-docetaxel and total impurities.

| **Compositions comprising Docetaxel, ethanol absolute and polysorbate 80 standard grades (example 2) or non-standard grades (example 1)** | | | |
|---|---|---|---|
| **Treatment** | **Type of Polysorbate 80** | **7-Epidoxetaxel [%]** | **Total impurities [%]** |
| No thermal treatment | Polysorbate 80 standard grade | 0.10 | 0.10 |
| | Polysorbate 80 non-standard grade | 0.09 | 0.09 |
| Heating at 70° C/30 min. | Polysorbate 80 standard grade | 4.07 | 4.17 |
| | Polysorbate 80 non-standard grade | 2.28 | 2.38 |
| Heating at 70°C/120 min. | Polysorbate 80 standard grade | 16.3 | 17.1 |
| | Polysorbate 80 non-standard grade | 9.01 | 9.21 |

### Chromatographic conditions

Column: Inertsil C8 (250 mm x 4.6, 5 µm)
Flow rate: 1 ml/min
Temperature: room temperature
Wavelength: 231 nm
Injection volume: 20 µl
Mobile phase : H₂O : Acetonitrile (50:50)
LOQ: 0.08%
Retention time for Docetaxel: 12.6 minutes.,
Retention time for 7 -Epi-docetaxel: RRT approx 1.62
Diluent: Acetonitrile

Standard preparation: Weight approximately 20 mg of Docetaxel reference standard and transfer it to a volumetric flask of 25 ml. Dissolve the reference standard with diluent and fill up to volume (Concentration: 0.8 mg/ml).

Standard 1%: Take 1 ml of the standard solution and make a dilution to 100 ml with diluent (Concentration: 0.008 mg/ml).

Sample solution for assay and purity (Docetaxel 20 mg): Weight the vial with the product and without the crimp cap and then transfer the content, with help of diluent, to a volumetric vessel of 25 ml. Wash the vial several times and fill to volume with the diluent. Wash the empty vial and dry it. Weight again and calculate the mass by difference (Theoretical concentration: 0.8 mg Docetaxel/ml).

Sample solution for assay and purity (Docetaxel 80 mg): Weight the vial with the product and without the crimp cap and then transfer the content, with help of diluent, to a volumetric flask of 100 ml. Wash the vial several times and fill to volume with the diluent. Wash the empty vial and dry it. Weight again and calculate the mass by difference. (Theoretical concentration: 0.8 mg Docetaxel/ml).

### Example 4: Compositions according to the invention containing citric acid

Dissolve 180 mg of anhydrous citric acid in approximately 30 ml of ethanol absolute by stirring at ambient temperature. Add 1.8 g of Docetaxel and stir until complete dissolution. Add 46.8 g of polysorbate 80 (Crillet 4 HP or Crillet NF) to the solution and stir to get a homogenous solution. Fill up to 90 ml with ethanol absolute and mix. Filter the solution and fill the solution into vials. Flush the vials with nitrogen. Close the vials with flouropolymer coated rubber stoppers and seal them with crimp caps.

| **Compositions comprising Docetaxel, polysorbate 80 standard grade, citric acid and ethanol absolute** | | | |
|---|---|---|---|
| **Treatment** | **Concentration of Citric Acid [mg/ml]** | **7-Epidoxetaxel [%]** | **Total impurities [%]** |
| No thermal treatment | No Citric Acid | 0.10 | 0.10 |
| | 0.5 | 0.11 | 0.11 |
| | 1.0 | - | - |
| | 2.0 | - | - |
| Heating at 70° C/30 min. | No Citric Acid | 4.07 | 4.17 |
| | 0.5 | 0.30 | 0.41 |
| | 1.0 | 0.09 | 0.20 |
| | 2.0 | 0.09 | 0.19 |
| Heating at 70°C/120 min. | No Citric Acid | 16.30 | 17.14 |
| | 0.5 | 1.02 | 1.12 |
| | 1.0 | 0.26 | 0.47 |
| | 2.0 | 0.13 | 0.23 |

A minimum of 1.0 mg/ml citric acid, corresponding to a ratio of ratio 1:20 (mg citric acid : mg Docetaxel), is required to stabilize the composition when using standard grades of polysorbate 80.

The increase in stability was even more prominent when combining citric acid and polysorbate 80 non-standard grades within one composition.

| **Compositions comprising Docetaxel, polysorbate 80 non-standard grades, citric acid and ethanol absolute** | | | |
|---|---|---|---|
| **Treatment** | **Concentration of Citric Acid [mg/ml]** | **7-Epidoxetaxel [%]** | **Total impurities [%]** |
| No thermal treatment | No Citric Acid | 0.09 | 0.09 |
| | 0.5 | 0.10 | 0.10 |
| | 1.0 | - | - |
| | 2.0 | - | - |
| Heating at 70° C/30 min. | No Citric Acid | 2.28 | 2.38 |
| | 0.5 | 0.23 | 0.34 |
| | 1.0 | n. d. | 0.10 |
| | 2.0 | 0.10 | 0.11 |
| Heating at 70°C/120 min. | No Citric Acid | 9.01 | 9.21 |
| | 0.5 | 0.67 | 0.79 |
| | 1.0 | 0.20 | 0.39 |
| | 2.0 | 0.11 | 0.22 |

A minimum of 0.5 mg/ml citric acid, corresponding to a ratio of ratio 1:40 (mg citric acid : mg Docetaxel) is capable to stabilize the composition when using non-standard grades of polysorbate 80.

## Claims

1. Pharmaceutical composition comprising:
• a pharmaceutical acceptable taxane derivative of formula (I) 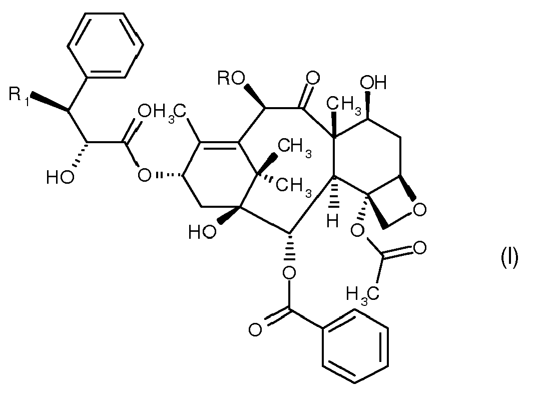 wherein R represents a hydrogen atom or an acetyl radical and R₁ represents a ter-butoxycarbonylamino or a benzoylamino radical, and
• a polysorbate 80 having a water content of less than 1.5% and a peroxide value of less than 5.

2. Pharmaceutical composition according to claim 1 wherein the polysorbate 80 has a water content of less than 0.5%, preferably less than 0.2%.

3. Pharmaceutical composition according to claim 1 or 2 wherein the polysorbate 80 has a peroxide value of less than 3, preferably less than 2.

4. Pharmaceutical composition according to any of the previous claims containing a physiologically acceptable solvent.

5. Pharmaceutical composition according to claim 4, wherein the solvent is selected in the group consisting of ethanol, DMSO, polyethylene glycol, glycerol, propanediol, polypropylene glycol, glycofurol, benzyl alcohol and N-methyl-2-pyrolidone.

6. Pharmaceutical composition according to claims 4 to 5, wherein the solvent is ethanol.

7. Pharmaceutical composition according to claims 4 to 6, wherein the solvent is ethanol anhydrous as defined in the European Pharmacopoeia 2007.

8. Pharmaceutical composition according to any of the previous claims, wherein said taxane derivative of formula I is selected from the group consisting of docetaxel and paclitaxel.

9. Pharmaceutical composition according to any of the previous claims, wherein said taxane derivative of formula I is docetaxel.

10. Pharmaceutical composition according to any of the previous claims, wherein docetaxel is in form of a pharmaceutically acceptable anhydrate, solvate and/or hydrate.

11. Pharmaceutical composition according to any of the previous claims, wherein the composition is presented in the form of a one compartment system or a two compartment system.

12. Pharmaceutical composition according to any of the previous claims, wherein the water content of the composition is less than about 1.5% by weight of the composition.

13. Pharmaceutical composition according to any of the previous claims, wherein the water content of the composition is less than about 0.5% by weight of the composition.

14. Pharmaceutical composition according to claims any of the previous claims containing an organic and/or and inorganic acid.

15. Pharmaceutical composition according to claim 14, wherein the acid is selected from the group consisting of citric acid, acetic acid, formic acid, ascorbic acid, aspartic acid, benzoic acid, hydrochloric acid, sulphuric acid, phosphoric acid, tartaric acid, glutamic acid, lactic acid, maleic acid or succinic acid.

16. Pharmaceutical composition according to claims 14 to 15, wherein the acid is citric acid.

17. Pharmaceutical composition according to claim 16, wherein citric acid is citric acid anhydrous as defined in the European Pharmacopoeia 2007.

18. Pharmaceutical composition according to claims 5 to 7 wherein the concentration of citric acid is from 0.5 to 2 mg/ml and/or where citric acid is added in a ratio (mg citric acid : mg Docetaxel) of from 1:40 to 1:10.
